# EUROPEAN PATENT SPECIFICATION

(11) **EP 1 793 862 B1**
(45) Date of publication and mention of the grant of the patent: **23.03.2011**
(21) Application number: 05774046.6
(22) Date of filing: 26.07.2005
(51) Int. Cl.: A61K 47/48

(54) **A PROCESS FOR THE PREPARATION OF A PIROXICAM: BETA-CYCLODEXTRIN INCLUSION COMPOUND**
VERFAHREN ZUR HERSTELLUNG EINER PIROXICAM:BETA-CYCLODEXTRIN-EINSCHLUSSVERBINDUNG
PROCEDE POUR LA PREPARATION DES COMPOSES D'INCLUSION PIROXICAM: BETA-CYCLODEXTRINE

(30) Priority: 02.08.2004 EP 04018261
(43) Date of publication of application: 13.06.2007
(73) Proprietor: CHIESI FARMACEUTICI S.p.A., 43100 Parma (IT)
(72) Inventor: PIGHI, Roberto, I-43100 Parma (IT); FJORDGAARD ANDERSEN, Søren, 43100 Parma (IT)
(74) Representative: Minoja, Fabrizio
(86) International application number: PCT/EP2005/008105
(87) International publication number: WO 2006/013039

(56) References cited:
- EP-A- 0 153 998
- EP-A2- 0 295 476
- WO-A-94/20091
- WO-A-03/043602
- WO-A-2006/011044
- SHAN-YANG L. ET AL: "Solid particulates of drug-b-cyclodextrin inclusion complexes directly prepared by a spray drying technique" INTERNATIONAL JOURNAL OF PHARMACEUTICS, vol. 56, 1989, pages 249-259, XP008071450 cited in the application
- ACERBI D. ET AL: "Biopharmaceutical optimisation of. beta.- cyclodextrin inclusion compounds." DRUG INVESTIGATION, vol. 2, 1990, pages 29-36, XP008007124 cited in the application
- PAVLOVA A V ET AL: "ANALYTICAL CHARACTERIZATION OF THE PIROXICAM-BETA-CYCLODEXTRIN INCLUSION COMPLEX" ANALYTICAL LABORATORY, SPEKTROTEH, SOFIA, BG, vol. 4, no. 2, 1995, pages 87-91, XP008007120 ISSN: 0861-4938 cited in the application
- REDENTI E. ET AL: "A study on the differentiation between amorphous piroxicam:beta cyclodextrin complex and a mixture of the two amorphous components" INTERNATIONAL JOURNAL OF PHARMACEUTICS, vol. 129, 1996, pages 289-294, XP008071451
- PEOA R ET AL: "OBTENTION AND CHARACTERIZATION OF DRUG/CYCLODEXTRIN COMPLEXES OBTAINED BY THE FREEZE-DRYING AND SPRAY-DRYING METHODS" INTERNATIONAL CONGRESS OF TECHNOLOGY AND PHARMACOLOGY, ASSOC. PHARM. GALENIQUE IND., CHATENAY MALABRY, FR, vol. 5, 1992, pages 331-338, XP008071559
- JUG M. ET AL: "Hydroxypropyl methylcellulose microspheres with piroxicam and piroxicam-hydroxypropyl-b-cyclodextrin inclusion complex" PHARMAZIE, vol. 59, 2004, pages 686-691, XP008071487
- VAN HEES T. ET AL: 'Application of supercritical carbon dioxide for the preparation of a piroxicam-[beta]-cyclodextrin inclusion compound' PHARMACEUTICAL RESEARCH vol. 16, no. 12, 01 December 1999, pages 1864 - 1870, XP001020308

## Description

The present invention relates to a process for the preparation of an inclusion compound of piroxicam with β-cyclodextrin in a molar ratio of 1 to 2.5 by spray-drying, applicable on a pilot or industrial scale.

More particularly, the present invention is directed to a process for the preparation of 1:2.5 piroxicam:β-cyclodextrin inclusion compound provided with optimal physico-chemical characteristics as well as technological and biopharmaceutical properties for preparing solid pharmaceutical compositions for the oral administration.

### BACKGROUND OF THE INVENTION

Piroxicam is a compound belonging to the class of the Non Steroidal Anti-Inflammatory Drugs (NSAIDs) widely applied in rheumatoid arthritis, osteoarthritis, acute pain in musculoskeletal disorders, post-operative and post-traumatic pain and dysmenorrhoea.

Piroxicam is poorly soluble in water (0.003% at pH 5, 37°C) and exhibits a low surface wettability (water contact angle 76°) and a high crystal lattice energy as demonstrated by its melting point (198-200°C).

Since piroxicam molecule exhibits good membrane permeation characteristics, its low solubility is responsible for the slow dissolution rate in the gastro-intestinal fluids, which in turn results in slow absorption and delay in the onset of action.

Slow dissolution can also exacerbate local side effects associated to the drug (e.g. gastric irritation).

The handling of piroxicam is complicated due to its possible tautomeric switches and polymorphism. Said molecule can indeed exist in two polymorphic forms α and β, which have the same intramolecular structure
EZE (I) but different intra- and intermolecular hydrogen bond interactions and in the pseudopolymorph which is the hydrate of the zwitter-ionic form ZZZ, one of the possible resonance forms of which is represented by formula (II)

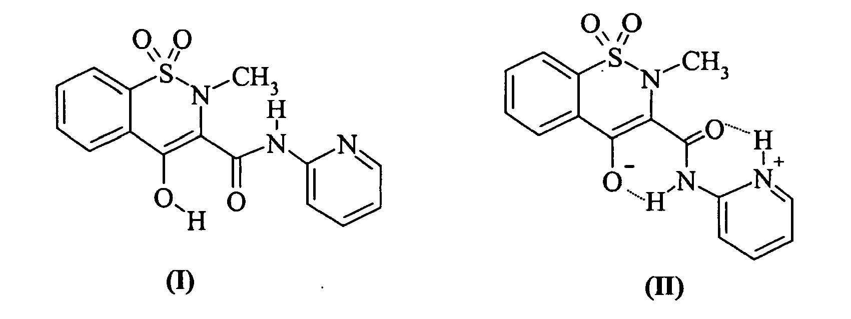

An efficient method for overcoming the problems related to the low solubility of piroxicam relies on the preparation of inclusion complexes with cyclodextrins as claimed in EP 153998. Hereinafter, the terms complexes, inclusion complexes and inclusion compounds are used as synonyms.

Cyclodextrins (CDs) are natural cyclic oligosaccharides having a torus-like macro-ring shape obtained by enzymatic degradation of starch. The three major cyclodextrins consist of 6(α), 7(β) or 8(γ) (1→4) D-glucopyranosidic units. Among them, βCD turned out to be the most useful for complexing piroxicam.

The solubilisation kinetic in water of piroxicam, released from the inclusion complex with β-cyclodextrin in the preferred molar ratio of 1 to 2.5, is the fastest of any other piroxicam obtained through any technological modification of the crystalline form known so far.

For solubilisation kinetic we mean the time to reach the highest concentration of dissolved piroxicam after dispersion in water of the inclusion complex in the form of a powder.

The 1:2.5 piroxicam:β-cyclodextrin inclusion complex (in the following 1:2.5 PβCD), having the molecular formula C₁₅H₁₃N₃O₄S*2.5 C₄₂H₇₀O₃₅ and a molecular weight of 3168.912, has been also referred to as CHF 1194.

The 1:2.5 PβCD inclusion compound, like piroxicam, exhibits anti-inflammatory, analgesic and antipyretic properties. As analgesic drug, it is indicated for the treatment of diseases such as dental pain, post-traumatic pain, headache and dysmenorrhoea. It is administered by oral route in the form of tablets or granules, preferably tablets.

Pre-clinical and clinical studies have demonstrated that the oral absorption of piroxicam, from 1:2.5 PβCD tablets and granules is faster and more efficient than that from piroxicam capsules.

In particular, the bioavailability of the active ingredient in terms of rate as well as of extent of absorption in the first two hours is significantly enhanced.

As a consequence of the faster absorption the onset of action of piroxicam from the 1:2.5 βCD complex is more rapid making the product particularly effective as analgesic. It has been observed that to ensure the best performances in terms of dissolution rate and hence rapid absorption of piroxicam following the administration of 1:2.5 PβCD tablets, the powdery raw material should be able to produce after dispersion in water a concentration of dissolved piroxicam equal to or higher than 0.4 g per 100 ml (0.4% w/v) within the first 15 minutes.

The successful results achieved with the use of cyclodextrins rely on the fact that, through complexation, it is possible to obtain a stable amorphous structure; since the amorphous form has a larger surface area and its lattice energy is much less than in crystals, both wettability and aqueous solubility of piroxicam are increased. Amorphous piroxicam as such is, indeed, a metastable form which crystallises within few hours. Moreover, it has also been demonstrated by Raman studies that piroxicam, in the β-cyclodextrin inclusion compound, assumes a zwitter-ionic structure with positive and negative charges delocalized similar to that of the hydrate pseudopolymorph (II). This structure is stabilized due to the chemical interaction with β-cyclodextrin via electrostatic and hydrogen bonds. The dipolar character of the zwitter-ionic structure improves the solubilisation kinetics and instant solubility of piroxicam.

Since the solubilisation kinetic in water of piroxicam also depends on the intramolecular structure assumed by piroxicam in the 1:2.5 PβCD inclusion compound, the relevant manufacturing process should be able to achieve the complete conversion of piroxicam in the zwitter-ionic form.

Moreover, the manufacturing process should be able to achieve the completeness of the inclusion reaction as well as the complete amorphization of the whole product.

On the other hand, an amorphous active substance may incur the risk of crystallisation during storage due to the presence of residual water. Once the amorphous substance is formulated under the form of solid pharmaceutical preparations such as tablets, said crystallization can be responsible for phenomena such as swelling or loss of hardness of the tablets. Therefore it is also very important that the manufacturing process yields an amorphous substance wherein the amount of residual water is the lowest as possible and in the case of 1:2.5 PβCD equal to or lower than 5% w/w, preferably equal to or lower than 4% w/w.

In summary, a manufacturing process suitable for the preparation of the 1:2.5 PβCD inclusion compound in the form of powder should be able to give rise to:
i) no significant degradation of the two ingredient, i.e. piroxicam and β-cyclodextrin;
ii) completeness of the inclusion reaction;
iii) complete amorphization;
iv) complete conversion of piroxicam into the zwitter-ionic form;
v) an amount of residual water equal to or lower than 5% w/w, preferably equal to or lower than 4% w/w.

Moreover said process should provide a 1:2.5 PβCD able to produce, after dispersion of the powder in water, a concentration of dissolved piroxicam equal to or higher than 0.4 g per 100 ml (0.4% w/v) within the first 15 minutes.

As reported above, the latter characteristic, when 1:2.5 PβCD is utilised for the preparation of solid pharmaceutical formulations for oral administration, and in particular tablets, is of paramount importance for ensuring optimal performances in term of piroxicam dissolution rate.

### PRIOR ART

Cyclodextrin inclusion complexes can be prepared by reaction between the components in the solid state or semi-solid state or liquid state.

In the solid state method, the two components may be optionally screened to uniform particle size and thoroughly mixed whereafter they are ground in a high-energy mill with optional heating, screened and homogenized.

In the semi-solid state, the two components are kneaded in the presence of small amounts of a suitable solvent, and the resulting complex is oven dried, screened and homogenized.

The complex formation in the liquid state is accomplished, in general terms, by dissolving the cyclodextrin and the drug in a suitable solvent and subsequently isolating the solid state complex by crystallization, evaporation, spray-drying or freeze-drying (lyophilization).

In particular freeze-drying and spray-drying are methods applicable on industrial scale.

Freeze-drying is the process of removing water from a product by sublimation, i.e. at a product temperature that is lower than its eutectic temperature.

In WO 03/105906 the applicant described a process for the preparation of 1:2.5 PβCD by freeze-drying on an industrial scale wherein a diluted aqueous solution of two components, piroxicam and β-cyclodextrin is subjected, before drying, to a freezing process at a very high rate.

Although it is very convenient for potentially thermolabile molecules such as piroxicam and β-cyclodextrin since it does not envision heating, freeze-drying involves a rather length step of removing a large amount of water by sublimation.

Spray-drying can constitute an alternative process of removing water from a product. It can be faster than freeze-drying but it requires heating so it could present some drawbacks when applied to potentially thermolabile molecules such as piroxicam and β-cyclodextrin.

Basically spray-drying is accomplished by atomizing a pre-heated solution (preferably an aqueous solution) into the drying chamber of the spray-drier apparatus where the small droplets are subjected to a stream of temperature-controlled hot gas and converted to powder particles. As the powder is discharged for the drying chamber, it is passed through a powder/gas separator, for example a cyclone, where it is further dried and collected.

The parameters which can be adjusted for obtaining a powder with well defined characteristics are: i) the type of atomizing device; ii) the temperature of the inlet gas used to dry the sprayed material in the drying chamber (hereinafter referred to as inlet temperature); iii) the gas flow rate and iv) the flow rate of the feed solution (hereinafter the feed flow rate). Another important parameter which affect the final moisture content of the powder is the temperature of the drying gas coming out from the spray-drying chamber (hereinafter referred to as outlet temperature).

The preparation of inclusion complexes of piroxicam with cyclodextrins by spray-drying on a lab scale is mentioned in several documents of the prior art. But the experimental conditions are not disclosed or, when disclosed, came out not suitable for preparing a 1:2.5 PβCD inclusion compound which fulfills the requirements previously illustrated.

EP 153998 generically discloses that complexes of piroxicam and cyclodextrins in a molar ratio comprised between 1:1 and 1:10 can be prepared in different ways:
a) by crystallization from an aqueous or an organic/aqueous solution containing the two ingredients;
b) by evaporation of a water/ammonia solution;
c) by freeze-drying or atomization in air stream (spray drying) of a water/ammonia solution.

All the examples refer to preparations of 1:2.5 PβCD on a lab scale (from milligram to grams). The conditions for obtaining the product by spray-drying are not reported.

EP 449167 discloses a process for preparing inclusion complexes of piroxicam with β-cyclodextrin characterized in that the two ingredients, both in powder form, are mixed together, then co-ground in a high-energy mill whose grinding chamber has been saturated with steam. In the example 2 of EP 449167, the dissolution rate of tablets containing as active ingredient the 1:2.5 PβCD prepared according to the claimed process was compared with that of analogous pharmaceutical composition containing the same active ingredient obtained by different methods, including spray drying and with a piroxicam composition in the form of capsules available on the market. The conditions for obtaining the product by spray-drying are not reported.

In Acerbi D et al (Drug Invest 1990, 2, Suppl. 4, 29-36), a flow-chart showing the manufacturing process(es) for 1:2.5 PβCD is sketched. As far as spray-drying process is concerned, no conditions are reported except for the temperature of the βCD aqueous solution (65°C-70°C) before it is added to the apparatus. Moreover, as it can be appreciated from Figure 7 dealing with the solubilisation curves of piroxicam from complexes prepared with different methods, 1:2.5 PβCD obtained by spray-drying, after dispersion of the powder, gives rise to a maximum concentration of dissolved piroxicam of only about 0.03 g per 100 ml of water within the first five minutes (0.03% w/v).

Pezoa R et al (Proceedings of the 6th International Conference on Pharmaceutical Technology, June, 2-4, 1992, Paris) reports the characterization of 1:1 PβCD complex obtained by freeze-drying and spray-drying methods. Experimental conditions are reported neither for freeze-drying nor for spray-drying. In the paper, it is generically stated that the dissolution profile of hard gelatine capsules containing the spray-dried complex shows a significant increase in the rate of dissolution but less than those containing the freeze-dried complex.

Pavlova A V et al (Analyt Lab 1995, 4, 87-91) concerns the analytical characterization of 1:2.5 PβCD inclusion complexes prepared in different ways. Among other methods, the inclusion complex was prepared by spray-drying but no conditions are reported.

In Van Hees T et al (Proceeding of the Ninth International Symposium on Cyclodextrins, Kluwer, 1999, 211-214), a comparative study of the dissolution properties of inclusion complexes of piroxicam with β-cyclodextrin prepared by different methods is reported. Complexes were prepared by supercritical CO₂, freeze-drying and spray-drying. The conditions for obtaining the product by spray-drying are not reported. The dissolution or solubilisation kinetic was determined on the spray-dried substance in a USP XXIII N. 2 dissolution apparatus using 500 ml of solutions at pH 1.2 and pH 6.8. Within the first 15 min, very low amounts of piroxicam, of about 30 mg and about 50 mg per 100 ml, were dissolved corresponding to concentrations of 0.03% and 0.05% w/v.

In Kata M et al (Proceedings of the 10th International Cyclodextrin Symposium, Wacker, 2000, 629-634) inclusion compounds of piroxicam: β-cyclodextrin in four different molar ratios (2:1, 1:1, 1:2 and 1:3) were prepared by spray-drying using a lab scale apparatus (Niro Minor atomizer). The powder mixtures were dissolved in dimethylformamide and water and submitted to spray-drying under the following conditions: feed flow rate: 600 ml/h (i.e. 0.6 1/h), temperature of inlet air: 155°C; temperature of outlet air: 90°C; pressure: 3 atm (corresponding to about 3 bar).

In Lin S-Y et al (Int J Pharm 1989, 56. 249-259) a 1:1 PβCD was prepared by spray-drying under the following conditions: inlet temperature: 145 ± 1°C, outlet temperature: 75± 1°C, drying (gas) flow rate: 0.37 m³/min (i.e. approximately 22 kg/h), atomizing air pressure: 1.0 kg/cm² (i.e. approximately 1 bar), sample feeding speed (feed flow rate): 4.5 ml/min (i.e. 0.27 1/h). The product turned out to be amorphous. The dissolution rate of the tablets prepared with the spray-dried product, carried out using the USP XXI paddle dissolution method at a rotational speed of 50 r.p.m. and at a temperature of 37°C, was faster than that of the physical mixture and pure drug but, as it can be appreciated from Figure 6(A) only about 20% of the amount of piroxicam was released after 30 minutes.

In Kata M and Lin S-Y processes, the outlet temperature is of 90°C or less.

As reported on page 4 of the present application, the 1:2.5 PβCD solid material to be utilised for the preparation of pharmaceutical formulations such as tablets should have a residual water equal to or lower than 5% w/w.

According to the findings of the applicant, outlet temperatures such as those reported in the above mentioned papers are too low for obtaining a product meeting said specification.

Therefore both Kata M and Lin S-Y disclose conditions not suitable for preparing 1:2.5 PβCD which fulfills the requirements previously mentioned in terms of residual water and solubilisation kinetic.

Van Hees T et al Pharm Res 1999, 16, 1864-1870 deals with the application of supercritical carbon dioxide for the preparation of a 1:2.5 PβCD inclusion compound. For comparison 1:2.5 PβCD was also prepared using the lab scale apparatus "Niro mobile minor™ spray-dryer", by applying the following conditions: inlet temperature of 175°C, feed flow rate of 15 ml/min (i.e. 0.9 1/h) and spray pressure of 0.2-0.3 MPa (corresponding to 2-3 bar).

In Table II, said spray-dried 1:2.5 PβCD turned out to include an amount of water of 4.4%. However, the paper is silent about the gas flow rate and the outlet temperature, that are parameters of paramount importance in order to obtain a 1:2.5 PβCD complex capable to ensure the desired solubilisation kinetic of piroxicam.

In view of the prior art, it would be highly advantageous to provide a process for preparing 1:2.5 PβCD inclusion compound by spray-drying, applicable on a pilot or an industrial scale, said process being able to give rise to:
i) no significant degradation of the two ingredient, i.e. piroxicam and β-cyclodextrin;
ii) completeness of the inclusion reaction;
iii) complete amorphization;
iv) complete conversion of piroxicam into the zwitter-ionic form;
v) an amount of residual water equal to or lower than 5% w/w, preferably equal to or lower than 4% w/w.

Moreover it would be even more advantageous to provide a spray-drying process which yields a 1:2.5 PβCD inclusion compound able to produce, after dispersion of the powder in water, a concentration of dissolved piroxicam equal to or higher than 0.4 g per 100 ml (0.4% w/v) within the first 15 minutes.

### THE OBJECT OF THE INVENTION

The present invention is directed to a process for the preparation of a 1:2.5 piroxicam:β-cyclodextrin (1:2.5 PβCD) inclusion compound by spray-drying, said process comprising the following steps:
i. dissolving piroxicam and β-cyclodextrin in the 1 to 2.5 molar ratio in water brought to a temperature higher than 60°C in the presence of ammonium hydroxide;
ii. feeding the resulting aqueous solution into the drying chamber of a spray-drier through an atomizing device to form droplets;
iii. introducing a stream of pre-heated drying gas into the drying chamber to form powder particles;
iv. further drying and separating the powder particles from the moist gas
characterized in that in step iii) the temperature of the inlet drying gas (inlet temperature) is comprised between 165°C and 200°C and the temperature of the outlet drying gas (outlet temperature) is comprised between 105°C and 130°C.

We have found that in order to obtain a 1:2.5 PβCD inclusion compound which fulfills the requirements mentioned before, it is necessary to strictly control both the inlet and the outlet temperatures in the drying chamber.

In particular, we have found that by applying an inlet temperature higher than 200°C, it is not possible to achieve after dispersion of the 1:2.5 PβCD powder in water a concentration of dissolved piroxicam equal to or higher than 0.4 g per 100 ml within the first 15 minutes. On the other hand, we have also found that if the outlet temperature is lower than 105°C, it would not be possible to obtain a residual amount of water lower than 5% w/w. Therefore the inlet temperature should be set to a value of at least 165°C and, after suitably adjusting other parameters such as the flow rate of the feed aqueous solution and the gas flow rate, the outlet temperature in the drying chamber should be equal to or higher than the critical value of 105°C.

By operating in the ranges of temperatures of the invention, piroxicam remains chemically stable and no significant degradation products of 1:2.5 PβCD were observed.

### DETAILED DESCRIPTION OF THE INVENTION

The characteristics of the process of the invention for preparing 1:2.5 PβCD inclusion compound on a pilot or industrial scale by spray-drying will be more apparent from the following detailed description.

For pilot or industrial scale, we mean the preparation of batches of at least 10 kg, preferably from 10 kg to 300 kg.

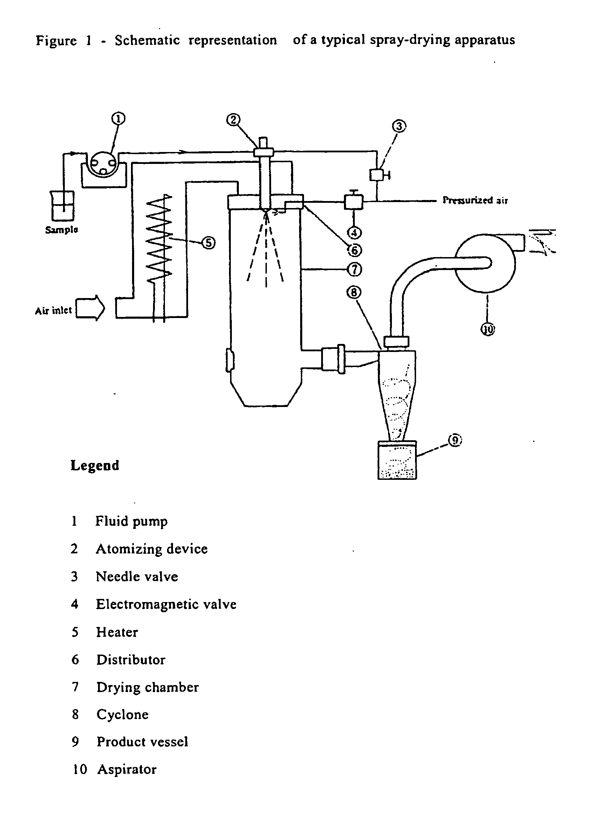

Spray-drier apparatus in a wide variety of sizes and configurations can be used as currently supplied by commercially suppliers. The diagram in Figure 1 shows a schematic representation of a typical spray-drying apparatus.

In a first step, piroxicam and β-cyclodextrin in the 1:2.5 molar ratio and ammonium hydroxide are added to a tank containing water brought to a temperature higher than 60°C, preferably higher than 70°C, more preferably between 70°C and 80°C, then mixed until dissolution. Advantageously the concentration of piroxicam in water shall be of about 2% w/v and that of β-cyclodextrin shall be of about 17% w/v. Advantageously concentrated ammonium hydroxide is added, preferably in a conc. of 28-30% w/w and in a 1:1 1 ratio w/w with respect to piroxicam.

In a second step the hot solution is loaded with a fluid pump (1 in Figure 1) through an atomizing device (2) into the drying chamber (7) of the spray-drier.

In the Example of the present invention, a pressure atomizing device was used and the process parameters of the spray-dryer were adjusted accordingly, in order to achieve an outlet temperature comprised between 105°C and 130°C.

The pressure atomizing device can consist of a single or a plurality of nozzles through which the solution is forced by the pump breaking up into droplets. When a pressure atomizing device consisting of a single nozzle is used, advantageously the pressure of the nozzle will be comprised between 10 and 350 bar, preferably between 20 and 200 bar. Typically, the nozzle shall have an internal diameter of 0.5 to 0.7 mm.

For the spraying process, other kinds of atomizing device such as a rotary (centrifugal) atomizing device or other suitable devices can be used. The person skilled in the art will adapt the various process conditions and parameters accordingly.

The rotary (centrifugal) atomizing device, for example, is a spinning disk assembly with radial or curved plates which rotates at high velocities, usually comprised between 15000 and 25000 r.p.m. The solution is delivered near the center and spreads between the two plates and is accelerated to high linear velocities before it is thrown off the disk in the form of droplets.

In the drying chamber, the droplets meet a stream of hot gas and they loose their moisture very rapidly while still suspended in the drying gas. While no particular restrictions are placed on the gas used to dry the sprayed solution, it is advantageous to use air, nitrogen gas or an inert gas, preferably air, more preferably with a residual moisture content equal to or lower than 7000 p.p.m. The gas is electrically heated (5) and can be introduced via a suitable distributor (6).

The heated gas stream may flow concurrently with the droplets, but it would also be possible to employ counter-current flow, cross-current flow, or other flow patterns.

Advantageously the inlet temperature in the drying chamber of the spray-drier will vary between 165°C and 200°C, more advantageously between 170°C and 200°C, preferably between 175°C and 195°C, more preferably between 178°C and 182°C.

The outlet temperature in the drying chamber shall be comprised between 105°C and 130°C, preferably between 110°C and 120°C, even more preferably between 112°C and 115°C.

In order to prepare an amount of inclusion complex of about 10 kg, the spray-drying process of the invention is carried out by applying a feed flow rate of at least 12 kg/h (approximately 12 1/h). For a higher amount, the feed flow rate shall be comprised between 12 kg/h and 200 ton/h, preferably between 12 kg/h and 300 kg/h.

Analogously, the flow rate of the drying gas is of at least 80 Kg/h, preferably 100 Kg/h, more preferably 300 Kg/h, even more preferably of at least 600 kg/h.

For higher amounts, the gas flow rate shall be comprised between 600 kg/h and 30 ton/h.

Once defined the inlet and outlet temperature ranges provided by the present invention, the other process parameters shall be properly and mutually adjusted by the person skilled in the art on the basis of the size of the batch.

In the example that follows, for a batch of about 10 kg of 1:2.5 PβCD, an inlet temperature of between 178°C and 182°C, a nozzle of 0.5 mm with a pressure of 21 bar, a feed flow of 12 kg/h (approximately 12 1/h) and a air flow rate of 600 kg/h are used in order to achieve the suitable outlet temperature of 112°C-115°C.

Advantageously the difference between the inlet and the outlet temperatures is comprised between 45°C and 95°C, preferably between 65°C and 75°C.

The powder is dried and separated from the moist gas in a cyclone (8) by centrifugal action. The centrifugal action is caused by the great increase in gas speed when the mixture of powder particles and gas enters into the cyclone. The dense powder particles are forced toward the cyclone walls and the product is collected under the cyclone on a vessel (9) through a discharging device such as a rotary valve. The lighter particles of moist gas are aspirated away by an aspirator (10) through the exhaust pipes.

Alternatively, separation may be achieved by using a filter medium such as a membrane medium (bag filter), a sintered metal fiber filter, or the like.

In the amorphous 1:2.5 PβCD inclusion compound obtainable by the process of the present invention piroxicam is completely present in the zwitter-ionic form and it can be characterized by its Raman spectrum, X-ray powder diffraction pattern and thermal behavior which are reported in the PCT application n. WO 03/105906.

The FT-Raman spectrum, obtained by simply packing the powder into a cup, shows the following main peaks in the 1650-1000 cm⁻¹ range (accuracy ± 1 cm⁻¹):
1613 cm⁻¹ (sh), 1593 (s), 1578 (sh), 1561 (w), 1525 (br), 1519 (br), 1464 (m), 1436 (m), 1394 (s), 1331 (brm)/1306 (sh), 1280 (w), 1260 (w), 1234 (w), 1217 (vw), 1186 (w), 1158(m), 1119 (m), 1083 (w), 1053 (w), 1036 (w), 992 (w), 947 (brw).
Legend: sh = shoulder; s = strong; m = medium; w = weak; vw = very weak; br = broad, brm = broad medium, brw = broad weak.

The amount of residual water in the 1:2.5 PβCD obtainable by the process of the present invention can be determined by Karl-Fisher method and it should be equal or lower than 5% w/w, preferably equal to or lower than 4% w/w. Since 1:2.5 PβCD tends to absorb water, the determination should be carried out as soon as the product is obtained and in any case after protection from moisture ingress.

The solubilisation kinetic of piroxicam from the 1:2.5 PβCD shall be determined according to the dispersed powder method reported in the following Example 2.

Advantageously, the concentration of dissolved piroxicam within the first 15 minutes shall be equal to or higher than 0.4% w/v, preferably equal to or higher than 0.5% w/v.

The 1:2.5 PβCD obtainable with the process of the invention can be advantageously used to prepare pharmaceutical compositions having analgesic, anti-inflammatory and anti-rheumatic activity, for the oral administration, preferably in the form of tablets, effervescent tablets or sachets for oral administration, more preferably in the form of tablets.

Advantageously the tablets for oral administration contain between 50 mg and 200 mg of the 1:2.5 PβCD complex per unit dose, preferably 95.6 mg or 191.2 mg (corresponding to 10 and 20 mg of piroxicam, respectively) in admixture with suitable excipients such as lactose, crospovidone, sodium starch glycolate, silica, starch and magnesium stearate.

The following examples better illustrate the invention.

### Example 1 - Preparation of 1:2.5 PβCD by spray-drying

About 50 litres of water was poured into a tank and heated up to a temperature of 73°C-75°C.

8.6 kg (7.57 moles) of β-cyclodextrin, 1 kg (3.02 moles) of piroxicam and 1 kg of 28% ammonium hydroxide were added in succession, and the mixture stirred for 30 min. The solution was filtered using a 55 µm filter and loaded into a spray dryer Niro. The following process parameters were used: nozzle diameter: 0.5 mm; nozzle pressure: 21 bar; air flow rate: 600 kg/h; feed flow rate: 12 kg/h (approximately 12 1/h); inlet temperature: 182°C; outlet temperature: 113°C.

The 1:2.5 PβCD product in the form of free-flowing powder was collected under the cyclone through a rotary valve.

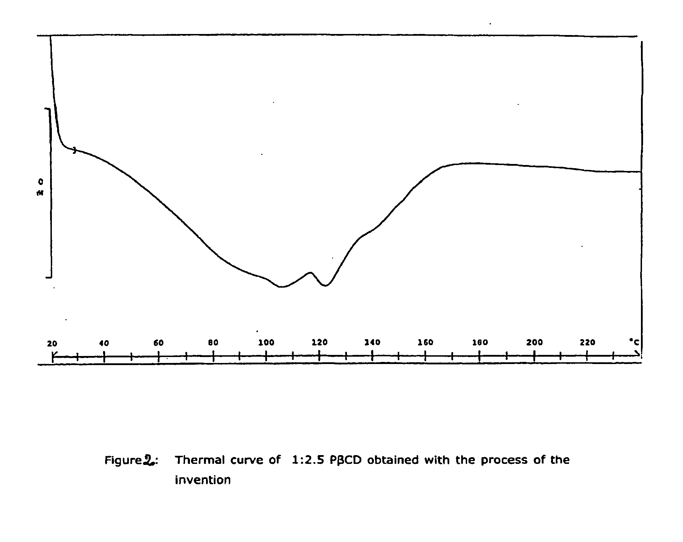

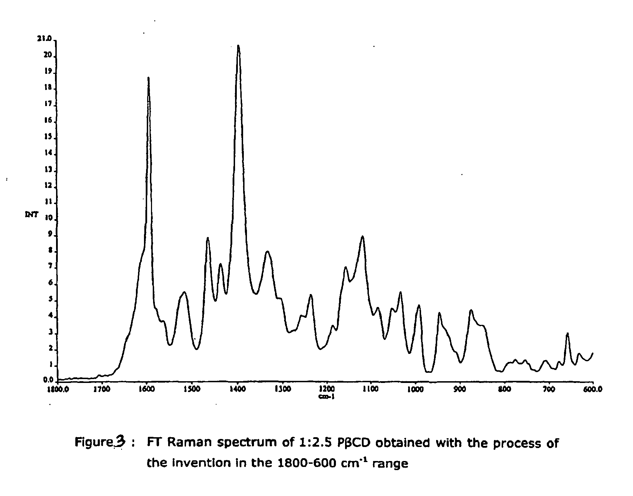

The resulting product shows the thermal curve and the Raman spectrum reported respectively in Figures 2 and 3, which are typical of a 1:2.5 PβCD wherein a complete inclusion complex reaction has occurred and piroxicam is present in the zwitter-ionic form. Powder X-ray analysis shows the diffused diffraction pattern typical of amorphous products. After HPLC analysis, no significant amount of degradation products of piroxicam was detected.

The residual amount of water was 3.8% w/w as determined by Karl Fischer method.

### Example 2 - Solubilisation kinetic of piroxicam from 1:2.5 PβCD prepared in the Example 1

The solubilisation kinetic was determined according to the dispersed powder method.

In a dissolution test apparatus Sotax A76, 250 ml of water were introduced and the temperature was set at 37°C ± 0.5°C. Then, 20 g of PβCD as obtained in the Example 1, corresponding to about 2 g of piroxicam, was added and the resulting dispersion was maintained under stirring at 125 r.p.m. After 15 minutes, an aliquot of the solution was withdrawn and filtered. The concentration of dissolved piroxicam, measured by UV spectrophotometry, turned out to be 0.5 g per 100 ml, i.e. 0.5% w/v.

## Claims

1. A process for the preparation of a 1:2.5 piroxicam:β-cyclodextrin inclusion compound comprising the following steps:
i. dissolving piroxicam and b-cyclodextrin in 1 to 2.5 molar ratio in water brought to a temperature higher than 60°C in the presence of ammonium hydroxide;
ii. feeding the resulting aqueous solution into the drying chamber of a spray-drier through an atomizing device to form droplets;
iii. introducing a stream of drying gas into the drying chamber to form powder particles;
iv. further drying and separating the powder particles from the moist gas;
**characterized in that** in step iii) the temperature of the inlet drying gas (inlet temperature) is comprised between 165°C and 200°C and the feed flow rate of the solution in step ii) and the flow rate of the drying gas are suitably adjusted in such a way as that the temperature of the outlet drying gas (outlet temperature) is comprised between 105°C and 130°C.

2. The process according to claim 1 wherein the feed flow rate of the solution of step ii) is of at least 12 kg/h.

3. The process according to claim 1 or 2 wherein the flow rate of the drying gas is of at least 600 kg/h.

4. The process according to any of claims 1 to 3, wherein the inlet temperature is comprised between 175°C and 195°C.

5. The process according to claim 4, wherein the inlet temperature is comprised between 178°C and 182°C.

6. The process according to any one of claims 1 to 5, wherein the outlet temperature is comprised between 110°C and 120°C.

7. The process according to claim 6, wherein the outlet temperature is comprised between 112°C and 115°C.

8. The process according to any of the claims 1 to 7 wherein the atomizing device is a rotary (centrifugal) atomizer.

9. The process according to any of claims 1 to 7 wherein the atomizing device is a pressure atomizer.

10. The process according to any of the preceding claims wherein the separation of the powder particles in step iv) is carried out in a cyclone by centrifugal action.

11. The process according to claim 1 wherein the water is brought to a temperature higher than 70°C

12. The process according to claim 1 wherein the water is brought to a temperature comprised between 70°C and 80°C.

13. The process according to claim 1 wherein that the ammonium hydroxide in step i) is used in a concentration ranging from 28 to 30 % and in a 1:1 ratio (w/w) with respect to piroxicam.

## Patentansprüche

1. Verfahren zur Herstellung einer 1:2,5 Piroxicam:β-Cyclodextrin-Einschlussverbindung, umfassend die folgenden Stufen:
i. Auflösen von Piroxicam und b-Cyclodextrin in einem molaren Verhältnis von 1 bis 2,5 in Wasser, welches auf eine Temperatur von mehr als 60°C gebracht worden ist, und zwar in Gegenwart von Ammoniumhydroxid;
ii. Zuführen der resultierenden wässrigen Lösung zu der Trockenkammer eines Sprühtrockners, und zwar durch eine Atomisiervorrichtung um Tröpfchen zu bilden;
iii. Einführen eines Stroms eines Trocknungsgases in die Trockenkammer um Pulverteilchen zu bilden;
iv. weiteres Trocknen und Abtrennen der Pulverteilchen von dem feuchten Gas;
**dadurch gekennzeichnet, dass** in der Stufe iii) die Temperatur des eingeführten Trocknungsgases (Einlasstemperatur) im Bereich zwischen 165°C und 200°C liegt und die Zufuhrgeschwindigkeit der Lösung in der Stufe ii) und die Fließgeschwindigkeit der Trocknungsgase in geeigneter Weise so eingestellt werden, dass die Temperatur des austretenden Trocknungsgases (Austrittstemperatur) im Bereich zwischen 105°C und 130°C liegt.

2. Verfahren nach Anspruch 1, wobei die Zufuhrgeschwindigkeit der Lösung in der Stufe ii) mindestens 12 kg/h beträgt.

3. Verfahren nach Anspruch 1 order 2, wobei die Strömungsgeschwindigkeit des Trocknungsgases mindestens 600 kg/h beträgt.

4. Verfahren nach einem der Ansprüche 1 bis 3, wobei die Einlasstemperatur im Bereich zwischen 175°C und 195°C liegt.

5. Verfahren nach Anspruch 4, wobei die Einlasstemperatur im Bereich zwischen 178°C und 182°C liegt.

6. Verfahren nach einem der Ansprüche 1 bis 5, wobei die Austrittstemperatur im Bereich zwischen 110°C und 120°C liegt.

7. Verfahren nach Anspruch 6, wobei die Austrittstrittstemperatur im Bereich zwischen 112°C und 115°C liegt.

8. Verfahren nach einem der Ansprüche 1 bis 7, wobei die A-tomisiervorrichtung ein rotierender (zentrifugaler) Atomisator ist.

9. Verfahren nach einem der Ansprüche 1 bis 7, wobei die A-tomisiervorrichtung ein Druckatomisierer ist.

10. Verfahren nach einem der voranstehenden Ansprüche, wobei die Abtrennung der Pulverteilchen in der Stufe iv) in einem Zyklon durch Zentrifugalwirkung durchgeführt wird.

11. Verfahren nach Anspruch 1, wobei das Wasser auf eine Temperatur von mehr als 70°C gebracht wird.

12. Verfahren nach Anspruch 1, wobei das Wasser auf eine Temperatur im Bereich zwischen 70°C und 80°C gebracht wird.

13. Verfahren nach Anspruch 1, wobei das Ammoniumhydroxid in der Stufe i) verwendet wird in einer Konzentration im Bereich von 28 bis 30%, und zwar in einem Verhältnis von 1:1 (w/w) bezüglich des Piroxicams.

## Revendications

1. Procédé pour la préparation d'un composé d'inclusion de 1:2,5 piroxicam:β-cyclodextrine comprenant les étapes suivantes :
i. dissolution de piroxicam et de b-cyclodextrine dans un rapport molaire de 1 à 2,5 dans de l'eau amenée à une température supérieure à 60 °C en présence d'hydroxyde d'ammonium ;
ii. fourniture de la solution aqueuse ainsi obtenue dans la chambre de séchage d'un sécheur atomiseur par l'intermédiaire d'un dispositif d'atomisation pour former des gouttelettes ;
iii. introduction d'un flux de gaz de séchage dans la chambre de séchage pour former des particules de poudre ;
iv. séchage supplémentaire et séparation des particules de poudre à partir du gaz humide ;
**caractérisé en ce que**, au cours de l'étape iii), la température du gaz de séchage à l'entrée (température d'entrée) est comprise entre 165 °C et 200 °C et le débit de fourniture de la solution au cours de l'étape ii) et le débit du gaz de séchage sont ajustés de telle sorte que la température du gaz de séchage en sortie (température de sortie) soit comprise entre 105°C et 130°C.

2. Procédé selon la revendication 1, dans lequel le débit de fourniture de la solution au cours de l'étape ii) est d'au moins 12 kg/h.

3. Procédé selon la revendication 1 ou 2, dans lequel le débit du gaz de séchage est d'au moins 600 kg/h.

4. Procédé selon l'une quelconque des revendications 1 à 3, dans lequel la température d'entrée est comprise entre 175 °C et 195 °C.

5. Procédé selon la revendication 4, dans lequel la température d'entrée est comprise entre 178 °C et 182 °C.

6. Procédé selon l'une quelconque des revendications 1 à 5, dans lequel la température de sortie est comprise entre 110 °C et 120 °C.

7. Procédé selon la revendication 6, dans lequel la température de sortie est comprise entre 112 °C et 115°C.

8. Procédé selon l'une quelconque des revendications 1 à 7, dans lequel le dispositif d'atomisation est un atomiseur rotatif (centrifuge).

9. Procédé selon l'une quelconque des revendications 1 à 7, dans lequel le dispositif d'atomisation est un atomiseur sous pression.

10. Procédé selon l'une quelconque des revendications précédentes, dans lequel la séparation des particules de poudre au cours de l'étape iv) est exécutée dans un cyclone par une action centrifuge.

11. Procédé selon la revendication 1, dans lequel l'eau est amenée à une température supérieure à 70 °C.

12. Procédé selon la revendication 1 dans lequel l'eau est amenée à une température comprise entre 70 °C et 80 °C.

13. Procédé selon la revendication 1 dans lequel au cours de l'étape i), l'hydroxyde d'ammonium est utilisé dans une concentration allant de 28 % à 30 % et dans un rapport de 1:1 (p/p) par rapport au piroxicam.
